# EUROPEAN PATENT APPLICATION

(11) **EP 3 904 521 A1**
(43) Date of publication of application: **03.11.2021**
(21) Application number: 19905042.8
(22) Date of filing: 18.12.2019
(51) Int. Cl.: C12N 15/70, C12N 15/77, C12N 15/67, C12P 13/04

(54) **E. COLI VARIANT STRAIN OR CORYNEBACTERIUM GLUTAMICUM VARIANT STRAIN PRODUCING L-AMINO ACIDS, AND METHOD FOR PRODUCING L-AMINO ACIDS USING SAME**

(30) Priority: 26.12.2018 KR 20180169847
(71) Applicant: Daesang Corporation, Seoul 02586 (KR)
(72) Inventor: LEE, Sun Hee, Yongin-si Gyeonggi-do 16923 (KR); KIM, Hyun Ho, Seoul 05236 (KR); KIM, Hyun Young, Yongin-si Gyeonggi-do 16867 (KR); JO, Young Il, Seoul 04995 (KR); KIM, Yong Soo, Suwon-si Gyeonggi-do 16694 (KR); YANG, Cheol Min, Goyang-si Gyeonggi-do 10474 (KR); SHIN, Won Joo, Incheon 21318 (KR)
(74) Representative: Nony
(86) International application number: PCT/KR2019/017934
(87) International publication number: WO 2020/138815

(57) **Abstract**

The present disclosure relates to an L-amino acid-producing *E. coli* mutant strain or *Corynebacterium glutamicum* mutant strain having enhanced L-amino acid productivity, and a method of producing L-amino acid using the same. The L-amino acid-producing *E. coli* mutant strain and *Corynebacterium glutamicum* mutant strain according to the present disclosure exhibit an enhanced ability to produce L-amino acid, such as L-tryptophan, L-phenylalanine, L-tyrosine, L-glutamine, L-lysine, L-arginine, L-valine, L-leucine, L-isoleucine, L-threonine, L-histidine, L-serine, or L-citrulline, compared to parent strains thereof, and are capable of producing a high concentration of L-amino acid in high yield.

## Description

### TECHNICAL FIELD

The present disclosure relates to an *E. coli* mutant strain or *Corynebacterium glutamicum* mutant strain having enhanced L-amino acid productivity and a method of producing L-amino acid using the same.

### BACKGROUND ART

L-tryptophan is one of the essential amino acids, and is required to polymerize proteins or synthesize vitamins such as nicotinic acid amide *in vivo,* and is widely used as an amino acid fortifying agent, a feed additive, a raw material for pharmaceuticals such as an infusion solution, and a health food material. L-phenylalanine is one of the essential amino acids, and serves *in vivo* as a precursor of: the non-essential amino acid tyrosine; a neurotransmitter such as dopamine, norepinephrine or adrenaline; or the skin pigment melanin, and is widely used as a raw material for food, cosmetics, and pharmaceuticals. In addition, various amino acids such as L-tyrosine, L-glutamine, L-lysine, L-arginine, L-valine, L-leucine, L-isoleucine, L-threonine, L-histidine, L-serine, and L-citrulline are widely used in various fields such as food, cosmetics and pharmaceuticals.

These L-amino acids were industrially produced by fermentation methods using naturally occurring microbial strains or their mutant strains modified to have enhanced L-amino acid productivity. As the mutant strains, auxotrophic strains or regulatory region mutant strains of microorganisms such as *Escherichia coli* and *Corynebacterium* have been used. Since the 1980s, as genetic recombination technology has rapidly developed and metabolic processes and their regulatory mechanisms have been identified in detail, many researchers have achieved great results by developing excellent recombinant strains using genetic manipulation techniques.

When genetic recombination technology is used, it is possible to enhance amino acid productivity by increasing the activity of an enzyme involved in amino acid biosynthesis or by inhibiting feedback caused by the produced L-amino acid. In addition, it is possible to enhance amino acid productivity by regulating the expression of amino acid exporter genes. US Patent No. 5,972,663 describes artificially overexpressing genes such as *mex, bmr* and *qacA* in several strains (such as *E. coli*) to enhance the abilities of the strains to produce L-cysteine, L-cystine, N-acetylserine and thiazolidine derivatives. European Patent No. 1016710 describes artificially overexpressing the genes *yahN, yeaS, yfiK* and *yggA* in an *Escherichia* sp. strain to enhance the ability of the strain to produce L-glutamic acid, L-lysine, L-threonine, L-alanine, L-histidine, L-proline, L-arginine, L-valine and L-isoleucine. Korean Patent No. 10-1023925 describes artificially overexpressing the gene *yddG* in an *Escherichia* sp. strain to enhance the ability of the strain to produce L-tryptophan and L-phenylalanine.

Based on these prior studies, the present inventors have constructed L-amino acid-producing mutant strains, which have enhanced ability to produce various L-amino acids, by regulating the expression of L-amino acid exporter genes in an *Escherichia* sp. strain and a *Corynebacterium* sp. strain, thereby completing the present disclosure.

### [Prior Art Documents]

### [Patent Documents]

(Patent Document 1) US Patent No. 5,972,663
(Patent Document 2) European Patent No. 1016710
(Patent Document 3) Korean Patent No. 10-1023925

### DISCLOSURE

### Technical Problem

In order to the above-described problems,
an object of the present disclosure is to provide an *Escherichia coli* mutant strain having enhanced L-amino acid productivity due to overexpression of at least one gene selected from the group consisting of *yicL, ydiN, ydhK, aaeB, yeeA, rhtC* and *emrD.*

Another object of the present disclosure is to provide a *Corynebacterium glutamicum* mutant strain having enhanced L-amino acid productivity due to overexpression of at least one gene selected from the group consisting of *yicL, ydiN, ydhK, aaeB, yeeA, rhtC* and *emrD.*

Still another object of the present disclosure is to provide a method for producing L-amino acid, the method comprising steps of: culturing the mutant strain in a medium; and recovering L-amino acid from the mutant strain or the medium.

### Technical Solution

An L-amino acid-producing mutant strain according to one embodiment of the present disclosure may be an *Escherichia coli* mutant strain or *Corynebacterium glutamicum* mutant strain having enhanced L-amino acid productivity due to overexpression of at least one gene selected from the group consisting of *yicL, ydiN, ydhK, aaeB, yeeA, rhtC* and *emrD.*

In the prior art, there was a case in which the production of the aromatic amino acids L-tryptophan and L-phenylalanine was improved by regulating the amino acid exporter gene *yddG* in an *Escherichia* sp. mutant strain so as to be overexpressed. However, the present inventors examined the effect of the gene *yddG* on the production of L-amino acids, and as a result, confirmed that L-tryptophan and L-phenylalanine were still expressed in the strain from which the gene *yddG* was deleted (see Experimental Example 1). Thereby, the present inventors recognized that genes other than the gene *yddG* are involved in the production of amino acids. In addition, the present inventors selected a new amino acid exporter gene and constructed L-amino acid-producing mutant strains having enhanced ability to produce L-amino acids such as L-tryptophan, L-phenylalanine, L-tyrosine, L-glutamine, L-lysine, L-arginine, L-valine, L-leucine, L-isoleucine, L-threonine, L-histidine, L-serine and L-citrulline.

According to one embodiment of the present disclosure, the amino acid exporter gene may be one of genes represented by the nucleotide sequences of SEQ ID NOs: 1 to 7. Specifically, the amino acid exporter gene may be the gene *yicL* represented by the nucleotide sequence of SEQ ID NO: 1, the gene *ydiN* represented by the nucleotide sequence of SEQ ID NO: 2, the gene *ydhK* represented by the nucleotide sequence of SEQ ID NO: 3 or 37, the gene *aaeB* represented by the nucleotide sequence of SEQ ID NO: 4, the gene *yeeA* represented by the nucleotide sequence of SEQ ID NO: 5, the gene *rhtC* represented by the nucleotide sequence of SEQ ID NO: 6, or the gene *emrD* represented by the nucleotide sequence of SEQ ID NO: 7 or 39.

In addition, the amino acid exporter gene may be one of genes encoding the proteins represented by the amino acid sequences of SEQ ID NOs: 8 to 14. Specifically, the amino acid exporter gene may be a *yicL* protein represented by the amino acid sequence of SEQ ID NO: 8, a *ydiN* protein represented by the amino acid sequence of SEQ ID NO: 9, a *ydhK* protein represented by the amino acid sequence of SEQ ID NO: 10 or 38, an *aaeB* protein represented by the amino acid sequence of SEQ ID NO: 11, a *yeeA* protein represented by the amino acid sequence of SEQ ID NO: 12, an *rhtC* protein represented by the amino acid sequence of SEQ ID NO: 13, or an *emrD* protein represented by the amino acid sequence of SEQ ID NO: 14.

According to one embodiment of the present disclosure, the mutant strain may be selected from an *Escherichia* sp. strain or a *Corynebacterium* sp. strain as a parent strain.

The *Escherichia* sp. strain is widely used for the production of several L-amino acids such as L-glutamic acid, L-lysine, L-threonine and L-cysteine, and is particularly known to have high L-tryptophan productivity. The parent strain is preferably *Escherichia coli,* which is easily available and has good convenience.

The *Corynebacterium* sp. strain has the advantage of low production of by-products while having high ability to produce L-amino acids such as L-glutamic acid and L-lysine, and is capable of stably producing amino acids even in a limited environment such as oxygen deficiency or sugar depletion. The parent strain is preferably *Corynebacterium glutamicum* which is mainly used to produce amino acids.

This parent strain may be a wild-type strain obtained in nature conditions or a strain in which the gene of the wild-type strain has been artificially manipulated.

In the present specification, the term "L-amino acid-producing *Escherichia coli* mutant strain" and "L-amino acid-producing *Corynebacterium glutamicum* mutant strain" refer to microorganisms mutated to have the ability to produce L-amino acids at a higher concentration than the parent strains. At this time, mutagenesis of the microorganisms may be performed by various means well known in the art, and one of physical or chemical mutagenesis methods may be used. For example, as chemical mutagens, N-methyl-N'-nitro-N-nitrosoguanidine (NTG), diepoxybutane, ethylmethane sulfonate, a mustard compound, hydrazine and nitrous acid may be used, but the chemical mutagens are not limited to these compounds. In addition, as physical mutagens, ultraviolet and gamma radiations may be used, but the physical mutagens are not limited thereto. Upon mutagenesis, the parent strain is affected by mutagens at a concentration sufficient to leave a surviving population having a certain size. The size may vary depending on the types of mutagenic factors, and depends on the amount of mutation that is induced in a surviving population at a constant killing rate by the mutagens. For example, when NTG is used, the killing rate may be 10% to 50% of the starting population. Mutagenesis by nitrous acid may be 0.01% to 0.1% of the starting population.

According to one embodiment of the present disclosure, the L-amino acids may be selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline, but are not limited thereto.

According to one embodiment of the present disclosure, the L-amino acid-producing *Escherichia coli* mutant strain and the L-amino acid-producing *Corynebacterium glutamicum* mutant strain may overexpress the amino acid exporter gene due to either transformation with the amino acid exporter gene or amplification of the number of copies of the amino acid exporter gene under the control of a strong promoter on the chromosome of each of the strains. According to one embodiment of the present disclosure, it is possible to obtain a mutant strain having enhanced L-amino acid productivity due to overexpression of the amino acid exporter gene compared to the parent strain, by inserting at least one of the seven amino acid exporter genes into a bacterial plasmid containing a promoter and introducing the plasmid into a parent strain.

Using this mutant strain, it is possible to produce a high concentration of L-amino acid in high yield. Specifically, a method for producing L-amino acid may comprise steps of: culturing the mutant strain in a medium; and recovering L-amino acid from the mutant strain or the medium.

In the present specification, the term "medium" refers to a medium which is used in culture of the *Escherichia coli* mutant strain and *Corynebacterium glutamicum* mutant strain of the present disclosure and contains carbon sources, nitrogen sources and inorganic salts so that the mutant strains are capable of producing a high yield of L-amino acid, for example, L-tryptophan, L-phenylalanine, L-tyrosine, L-glutamine, L-lysine, L-arginine, L-valine, L-leucine, L-isoleucine, L-threonine, L-histidine, L-serine or L-citrulline.

Examples of the carbon sources that may be used in the medium include, but are not limited to, saccharides and carbohydrates such as glucose, sugar, citrate, fructose, lactose, maltose or molasses; oils and fats, such as soybean oil, sunflower oil, castor oil or coconut oil; fatty acids such as palmitic acid, stearic acid or linoleic acid; glycerol; alcohols such as ethanol; and organic acids such as acetic acid. These substances may be used individually or as a mixture. Preferably, the medium for the *Escherichia coli* mutant strain may contain glucose. In addition, preferably, the medium for the *Corynebacterium glutamicum* mutant strain may contain glucose, molasses, or glucose and molasses. Most preferably, the medium may contain, as carbon sources, molasses in an amount of 5 to 60 parts by weight based on 100 parts by weight of glucose.

Examples of the nitrogen sources that may be used in the medium include, but are not limited to, peptone, meat extract, yeast extract, dried yeast, corn steep liquor, soybean cake, urea, thiourea, ammonium salt, nitrate, and other compounds organic or inorganic nitrogen. In addition, examples of inorganic salts that may be used in the medium include, but are not limited to, compounds containing magnesium, manganese, potassium, calcium, iron, zinc, cobalt, etc.

Meanwhile, examples of phosphorus sources that may be used in the medium include, but are not limited to, potassium dihydrogen phosphate or dipotassium hydrogen phosphate or the corresponding sodium-containing salts. In addition, the culture medium may contain metal salts such as magnesium sulfate or iron sulfate, which are required for growth. In addition, the above-described compounds may be used individually or as a mixture, but are not limited thereto.

Furthermore, in addition to the carbon source, nitrogen source and inorganic salt components, amino acids, vitamins, nucleic acids and compounds related thereto may be additionally added to the medium of the present disclosure. The above-described components may be added to the medium batchwise or in a continuous manner by a suitable method during culturing.

Meanwhile, the pH of the medium may be adjusted by adding a basic compound such as sodium hydroxide, potassium hydroxide or ammonia, or an acidic compound such as phosphoric acid or sulfuric acid in an appropriate manner. In addition, foaming may be suppressed using an anti-foaming agent such as a fatty acid polyglycol ester. To keep the medium in an aerobic condition, oxygen or an oxygen-containing gas (for example, air) may be injected into the medium.

In the present disclosure, the term "culturing" means growing a microorganism under artificially controlled suitable environmental conditions. Culturing of the microorganism of the present disclosure may be performed using the *Escherichia coli* or *Corynebacterium glutamicum* culturing method widely known in the art. Specifically, examples of the culturing method include, but are not limited to, batch culture, continuous culture, and fed-batch culture.

The temperature of the culturing may generally be, but not limited to, 20°C to 45°C. The culturing may be continued until the largest possible amount of an L-amino acid such as L-tryptophan, L-phenylalanine, L-tyrosine, L-glutamine, L-lysine, L-arginine, L-valine, L-leucine, L-isoleucine, L-threonine, L-histidine, L-serine or L-citrulline is produced. The culturing may be generally performed for, but not limited to, 10 to 160 hours. The produced L-amino acid may be released into the culture medium or contained in the mutant strain.

The method for recovering L-amino acid from the mutant strain or the medium in which the mutant strain has been cultured may be performed using various methods widely known in the art, for example, but not limited to, centrifugation, filtration, anion exchange chromatography, crystallization and HPLC.

### Advantageous Effects

The L-amino acid-producing *Escherichia coli* mutant strain or *Corynebacterium glutamicum* mutant strain according to the present disclosure may exhibit enhanced L-amino acid productivity compared to the parent strain, and may produce a high concentration of L-amino acid in high yield.

### Mode for Invention

Hereinafter, the L-amino acid-producing *Escherichia coli* mutant strain or *Corynebacterium glutamicum* mutant strain according to the present disclosure and the method of producing L-amino acid using the same will be described in more detail with reference to examples. However, this description is provided by way of example only to aid the understanding of the present disclosure, and the scope of the present disclosure is not limited by this illustrative description.

### Experimental Example 1-1. Construction of yddG Gene-Deleted Mutant Strains

In order to examine the effect of the *yddG* gene on L-amino acid production, *yddG* gene-deleted mutant strains were constructed.

The *yddG* gene-deleted mutant strains were constructed using an L-tryptophan-producing *E. coli* W0G strain (accession number: KFCC11660P) and an L-phenylalanine-producing *E. coli* MWTR42 strain (accession number: KFCC10780) as parent strains with reference to the experimental method described in the literature (One-step inactivation of chromosomal genes in Escherichia coli K-12 using PCR products, Datsenko KA, Wanner BL., Proc Natl Acad Sci USA, 2000 Jun 6; 97(12):6640-5).

First, to construct DNA fragments for disruption of the *yddG* gene, polymerase chain reaction (PCR) was performed using the chromosome of each parent strain and a pKD13 plasmid as a template and the primers shown in Table 1 below. At this time, the PCR was performed for a total of 30 cycles, each consisting of (i) denaturation at 95°C for 30 sec, (ii) annealing at 58°C for 30 sec, and (iii) extension at 72°C for 2 min. The PCR products were electrophoresed on 0.8% agarose gel, and bands of desired size were obtained. Using the fragments corresponding to the bands, overlap PCR was performed under the above-described PCR conditions, thereby constructing single DNA fragments for disruption of the *yddG* gene. The DNA fragments were transformed into the respective parent strains which were then streaked on kanamycin-containing solid media and cultured at 37°C for 24 hours. The resultant colonies were subjected to PCR under the above-described PCR conditions.

**[Table 1]**

| **Primer** | **Nucleotide sequence (5' → 3')** |
|---|---|
| yddG H1F (SEQ ID NO: 15) | CAATGCCGCTACTGTTGTTCCAGCC |
| yddG H1R (SEQ ID NO: 16) | CAGTGGTGCGTTTTTCTACCGCTAT |
| yddG H2F (SEQ ID NO: 17) | AATAACTGCCGGGTCTACGGCC |
| yddG H2R (SEQ ID NO: 18) | AACGTATTTTCTAAACGAATTTTAAACGGCG TC |
| yddG CF (SEQ ID NO: 19) | CGGAACAGTATGTGCAGGTGTTACGG |
| yddG CR (SEQ ID NO: 20) | AACAAACCAGTTACAACCACCGCAAC |

As a result, it was confirmed that the *yddG* gene was deleted in each colony.

In addition, the mutant strain obtained by deleting the *yddG* gene from the *E. coli* W0G strain was named W1G strain, and the mutant strain obtained by deleting the *yddG* gene from the *E. coli* MWTR42 strain was named MWTR5 strain.

### Experimental Example 1-2. Examination of L-tryptophan and L-phenylalanine Productivities of yddG Gene-Deleted Mutant Strains

The L-tryptophan productivities of the WOG and W1G strains and the L-phenylalanine productivities of the MWTR42 and MWTR5 strains were examined.

Each of the strains was 1% inoculated into a flask containing 10 ml of each medium having the composition shown in Table 2 below, and was cultured with shaking at 200 rpm at 37°C for 70 hours. Each culture was measured for absorbance at OD₆₁₀ₙₘ, and the amount of L-tryptophan or L-phenylalanine produced was compared between the strains. The results are shown in Table 3 below.

**[Table 2]**

| **Medium for tryptophan production** | | **Medium for phenylalanine production** | |
|---|---|---|---|
| **Component** | **Content** | **Component** | **Content** |
| Glucose | 80.0 g/L | Glucose | 80.0 g/L |
| (NH₄)₂SO₄ | 20.0 g/L | (NH₄)₂SO₄ | 20.0 g/L |
| K₂HPO₄ | 0.8 g/L | K₂HPO₄ | 1.0 g/L |
| K₂SO₄ | 0.4 g/L | KH₂PO₄ | 1.0 g/L |
| MgCl₂ | 0.8 g/L | K₂SO₄ | 0.4 g/L |
| Fumaric acid | 1.0 g/L | MgCl₂ | 1.0 g/L |
| Yeast extract | 1.0 g/L | Fumaric acid | 0.5 g/L |
| (NH₄)₆MO₇O₂₄ | 0.12 ppm | Yeast extract | 1.0 g/L |
| H₃BO₃ | 0.01 ppm | Glutamic acid | 0.5 g/L |
| CuSO₄ | 0.01 ppm | CaCl₂ | 5.00 ppm |
| MnCl₂ | 2.00 ppm | MnCl₂ | 2.00 ppm |
| ZnSO₄ | 0.01 ppm | ZnSO₄ | 1.00 ppm |
| CoCl₂ | 0.10 ppm | CoCl₂ | 0.10 ppm |
| FeCl₂ | 10.00 ppm | FeCl₂ | 10.00 ppm |
| Thiamine_HCl | 20.00 ppm | Thiamine_HCl | 20.00 ppm |
| L-tyrosine | 200.00 ppm | L-tyrosine | 200.00 ppm |
| L-phenylalanine | 300.00 ppm | CaCO₃ | 3 % |
| CaCO₃ | 3 % | - | - |

**[Table 3]**

| **Strain** | **L-tryptophan (g/L)** | **Strain** | **L-phenylalanine (g/L)** |
|---|---|---|---|
| W0G | 4.21±0.113 | MWTR42 | 21.3±0.115 |
| W1G | 2.53±0.132 | MWTR5 | 11.7±0.141 |

As shown in Table 3 above, it was confirmed that the *yddG*-gene mutant strains (W1G strain and MWTR5 strain) still produced L-tryptophan and L-phenylalanine, respectively. That is, it could be seen that, even if yddG known as an aromatic amino acid exporter gene was deleted, the L-amino acid productivity of each of the mutant strains was retained, indicating that genes involved in amino acid export are present in addition to *yddG.*

### Experimental Example 2-1. Construction of Mutant Strains Containing the Amplified Genes yicL, ydiN, ydhK, aaeB, yeeA, rhtC and emrD, Respectively

*E. coli* mutant strains into which *yicL, ydiN, ydhK, aaeB, yeeA, rhtC* and *emrD* genes have been inserted, respectively, were constructed.

As a parent strain, an L-tryptophan-producing *E. coli* W0G strain (accession number: KFCC 11660P) was used.

First, each of *yicL, ydiN, ydhK, aaeB, yeeA, rhtC* and *emrD* genes was amplified by PCR using the W0G strain as a template and the primers shown in Table 4 below, and then the PCR products were treated with the restriction enzymes *SacI* and *XbaI,* thereby preparing the genes.

As a result of sequencing, it was confirmed that the *yicL* gene has the nucleotide sequence of SEQ ID NO: 1, the *ydiN* gene has the nucleotide sequence of SEQ ID NO: 2, the *ydhK* gene has the nucleotide sequence of SEQ ID NO: 3 or 37, the *aaeB* gene has the nucleotide sequence of SEQ ID NO: 4, the *yeeA* gene has the nucleotide sequence of SEQ ID NO: 5, the *rhtC* gene has the nucleotide sequence of SEQ ID NO: 6, and the *emrD* gene has the nucleotide sequence of SEQ ID NO: 7 or 39.

The vector pTrc99A was digested by treatment with the restriction enzymes *SacI* and *XbaI,* and each of the prepared genes was inserted into the digested vector, thereby constructing the expression vectors pTrc99A::yicL, pTrc99A::ydiN, pTrc99A::ydhK, pTrc99A::aaeB, pTrc99A::yeeA, pTrc99A::rhtC, and pTrc99A::emrD. Each of the expression vectors was transformed into the W0G strain, thereby constructing the mutant strains W0G/pTrc99A::yicL, W0G/pTrc99A::ydiN, W0G/pTrc99A::ydhK, W0G/pTrc99A::aaeB, W0G/pTrc99A::yeeA, W0G/pTrc99A::rhtC, and W0G/pTrc99A::emrD, which contain each of the amplified genes.

**[Table 4]**

| **Primer** | **Nucleotide sequence (5' →3')** |
|---|---|
| yicL_F (SEQ ID NO: 21) | GCGAGCTCATGGGTTCCACCAGAAAGGG |
| yicL_R (SEQ ID NO: 22) | GCTCTAGATCACTTATGCCGCGCCGGA |
| ydiN_F (SEQ ID NO: 23) | GCGAGCTCATGTCTCAAAATAAGGCTTTCAGCA |
| ydiN_R (SEQ ID NO: 24) | GCTCTAGAGGCCATCAACCCAATCAATT |
| ydhK_F (SEQ ID NO: 25) | GCGAGCTCATGAACGCATCGTCATGGTCCTTGC |
| ydhK_R (SEQ ID NO: 26) | GCTCTAGATCACTTATGCCGCGCCGGA |
| aaeB_F (SEQ ID NO: 27) | GCGAGCTCATGGGTATTTTCTCCATTGCT |
| aaeB_R (SEQ ID NO: 28) | GCTCTAGATTTTGACTTAACTATCGGTca |
| yeeA_F (SEQ ID NO: 29) | GCGAGCTCGTGCGTGCCGATAAGTC |
| yeeA_R (SEQ ID NO: 30) | GCTCTAGATTATTTGCGCAAGGCCCG |
| rhtC_F (SEQ ID NO: 31) | GCGAGCTCATGTTGATGTTATTTCTCACCGT |
| rhtC_R (SEQ ID NO: 32) | gctctagaCTGGCATCACCGCGAAATAA |
| emrD_F (SEQ ID NO: 33) | GCGAGCTCATGAAAAGGCAAAGAAACG |
| emrD_R (SEQ ID NO: 34) | GCTCTAGACGGTGACGTGCGCTTAAAC |

### Experimental Example 2-2. Examination of L-tryptophan Productivities of Mutant Strains Containing the Amplified Genes yicL, ydiN, ydhK, aaeB, veeA, rhtC and emrD, Respectively

The L-tryptophan productivities of W0G/pTrc99A obtained by inserting only the vector pTrc99A into W0G, and the mutant strains WOG/pTrc99A::yicL, W0G/pTrc99A::ydiN, W0G/pTrc99A::ydhK, WOG/pTrc99A::aaeB, W0G/pTrc99A::yeeA, WOG/pTrc99A::rhtC, and WOG/pTrc99A::emrD, were examined.

Each of the strains was 1% inoculated into a flask containing 10 ml of each medium having the composition shown in Table 2 above, and was cultured with shaking at 200 rpm at 34°C for 72 hours. Each culture was measured for absorbance at OD₆₁₀ₙₘ, and the amount of L-tryptophan produced was compared between the strains. The results are shown in Table 5 below.

**[Table 5]**

| **Strain** | **L-tryptophan (g/L)** |
|---|---|
| W0G/pTrc99A (control) | 4.21±0.113 |
| WOG/pTrc99A::yicL | 4.83±0.102 |
| W0G/pTrc99 A: :ydiN | 4.86±0.169 |
| W0G/pTrc99 A: :ydhK | 4.88±0.133 |
| W0G/pTrc99A::aaeB | 4.91±0.101 |
| WOG/pTrc99A::yeeA | 4.95+0.123 |
| W0G/pTrc99A::rhtC | 5.02±0.131 |
| WOG/pTrc99A::emrD | 5.26±0.156 |

As shown in Table 5 above, it was confirmed that the L-tryptophan productivities of the *E. coli* mutant strains containing the amplified genes *yicL, ydiN, ydhK, aaeB, yeeA, rhtC* and *emrD,* respectively, increased compared to that of the control.

### Experimental Example 3-1. Construction of Mutant Strains Containing Amplified Genes yicL, ydiN, ydhK, aaeB, yeeA, rhtC and emrD, Respectively

*E. coli* mutant strains into which *yicL, ydiN, ydhK, aaeB, yeeA, rhtC* and *emrD* genes were inserted, respectively, were constructed.

Expression vectors were constructed in the same manner as in Experimental Example 2-1 above, except that an L-phenylalanine-producing *E. coli* MWTR42 strain (accession number: KFCC 10780) was used instead of the W0G strain. Each of the expression vectors was transformed into the MWTR42 strain, thereby constructing the mutant strains MWTR42/pTrc99A::yicL, MWTR42/pTrc99A::ydiN, MWTR42/pTrc99A::ydhK, MWTR42/pTrc99A::aaeB, MWTR42/pTrc99A::yeeA, MWTR42/pTrc99A::rhtC, and MWTR42/pTrc99A::emrD, which contain each of the amplified genes.

### Experimental Example 3-2. Examination of L-phenylalanine Productivities of Mutant Strains Containing the Amplified Genes yicL, ydiN, ydhK, aaeB, veeA, rhtC and emrD, Respectively

The L-phenylalanine productivities of MWTR42/pTrc99A obtained by inserting only the vector pTrc99A into MWTR42, and the mutant strains MWTR42/pTrc99A::yicL, MWTR42/pTrc99A::ydiN, MWTR42/pTrc99A::ydhK, MWTR42/pTrc99A::aaeB, MWTR42/pTrc99A::yeeA, MWTR42/pTrc99A::rhtC, and MWTR42/pTrc99A::emrD, were examined.

The amount of L-phenylalanine produced was compared between the strains in the same manner as in Experimental Example 2-2, and the results are shown in Table 6 below.

**[Table 6]**

| **Strain** | **L-phenylalanine (g/L)** |
|---|---|
| MWTR42/pTrc99A (control) | 21.3±0.115 |
| MWTR42/pTrc99A::yicL | 27.1±0.081 |
| MWTR42/pTrc99A::ydiN | 27.2±0.165 |
| MWTR42/pTrc99A::ydhK | 27.3±0.105 |
| MWTR42/pTrc99A::aaeB | 27.0±0.111 |
| MWTR42/pTrc99A: :yeeA | 27.3±0.103 |
| MWTR42/pTrc99A::rhtC | 26.7±0.122 |
| MWTR42/pTrc99A::emrD | 27.1±0.085 |

As shown in Table 6 below, it was confirmed that the L-phenylalanine productivities of the *E. coli* mutant strains containing the amplified genes *yicL, ydiN, ydhK, aaeB, yeeA, rhtC* and *emrD,* respectively, increased compared to that of the control.

### Experimental Example 4-1. Construction of Mutant Strains Containing the Amplified Genes yicL, ydiN, ydhK, aaeB, yeeA, rhtC and emrD, Respectively

*Corynebacterium glutamicum* mutant strains into which *yicL, ydiN, ydhK, aaeB, yeeA, rhtC* and *emrD* genes have been inserted, respectively, were constructed.

As a parent strain, L-tryptophan-producing *Corynebacterium glutamicum* DW28G (accession number: KTCT13769BP) was used.

First, each of *yicL, ydiN, ydhK, aaeB, yeeA, rhtC* and *emrD* genes was amplified by PCR using each of the expression vectors (pTrc99A::yicL, pTrc99A::ydiN, pTrc99A::ydhK, pTrc99A::aaeB, pTrc99A::yeeA, pTrc99A::rhtC, and pTrc99A::emrD) constructed in Experimental Examples 2-1 as a template and the primers shown in Table 7 below, and then the PCR products were 5'-phosphorylated, thereby preparing the genes. The vector pEKO was digested by treatment with the restriction enzyme *Eco53KI,* and each of the prepared genes was inserted into the digested vector, thereby constructing the expression vectors pEKO::yicL, pEKO::ydiN, pEKO::ydhK, pEKO::aaeB, pEKO::yeeA, pEKO::rhtC, and pEKO::emrD. Each of the expression vectors was transformed into the DW28G strain, thereby constructing the mutant strains DW28G/pEKO::yicL, DW28G/pEKO::ydiN, DW28G/pEKO::ydhK, DW28G/pEKO::aaeB, DW28G/pEKO::yeeA, DW28G/pEKO::rhtC, and DW28G/pEKO::emrD, which contain each of the amplified genes.

**[Table 7]**

| **Primer** | **Nucleotide sequence (5' →3')** |
|---|---|
| Primer-F (SEQ ID NO: 35) | gcgccgacatcataacggttctgg |
| Primer_R (SEQ ID NO: 36) | cgcaacgttcaaatccgctcccg |

### Experimental Example 4-2. Examination of L-tryptophan Productivities of Mutant Strains Containing the Amplified Genes yicL, ydiN, ydhK, aaeB, veeA, rhtC and emrD, Respectively

The L-tryptophan productivities of DW28G/pEKO obtained by inserting only the vector pEKO into DW28G, and the mutant strains DW28G/pEKO::yicL, DW28G/pEKO::ydiN, DW28G/pEKO::ydhK, DW28G/pEKO::aaeB, DW28G/pEKO::yeeA, DW28G/pEKO::rhtC, and DW28G/pEKO::emrD, were examined.

The amount of L-tryptophan produced was compared between the strains in the same manner as in Experimental Example 2-2, except that the composition shown in Table 8 was used as a medium. The results are shown in Table 9 below.

**[Table 8]**

| **Component** | **Content** |
|---|---|
| Cane molasses (glucose) | 100.00 g/L |
| KH₂PO₄ | 5.00 g/L |
| K₂HPO₄ | 5.00 g/L |
| K₂SO₄ | 0.40 g/L |
| MgSO₄ | 0.25 g/L |
| (NH₄)₂SO₄ | 20 g/L |
| Corn steep liquor | 10 g/L |
| CaCO₃ | 3% |

**[Table 9]**

| **Strain** | **L-tryptophan (g/L)** |
|---|---|
| DW28G/pEKO (control) | 2.64±0.107 |
| DW28G/pEKO::yicL | 3.57±0.115 |
| DW28G/pEKO: :ydiN | 3.05±0.089 |
| DW28G/pEKO: :ydhK | 3.11±0.095 |
| DW28G/pEKO::aaeB | 2.95±0.102 |
| DW28G/pEKO::yeeA | 3.21±0.113 |
| DW28G/pEKO::rhtC | 3.31±0.091 |
| DW28G/pEKO::emrD | 3.45±0.115 |

As shown in Table 9 above, it was confirmed that the L-tryptophan productivities of the *Corynebacterium glutamicum* mutant strains containing the amplified genes *yicL, ydiN, ydhK, aaeB, yeeA, rhtC* and *emrD**,*** respectively, increased compared to that of the control.

In conclusion, in the present disclosure, the *E. coli* mutant strain or *Corynebacterium glutamicum* mutant strain having enhanced L-tryptophan or L-phenylalanine productivity compared to the parent strain was obtained by inducing overexpression of the amino acid exporter gene *yicL, ydiN, ydhK, aaeB, yeeA, rhtC* or *emrD**,*** and it was confirmed that a high concentration of L-tryptophan or L-phenylalanine could be produced in high yield by using this mutant strain.

So far, the present disclosure has been described with reference to the embodiments thereof. Those of ordinary skill in the art to which the present disclosure pertains will appreciate that the present disclosure may be embodied in modified forms without departing from the essential characteristics of the present disclosure. Therefore, the disclosed embodiments should be considered from an illustrative point of view, not from a restrictive point of view. The scope of the present disclosure is defined by the claims rather than the foregoing description, and all differences within the scope equivalent thereto should be construed as being included in the present disclosure.

## Claims

1. A mutant strain having enhanced L-amino acid productivity due to overexpression of at least one gene selected from the group consisting of *yicL, ydiN, ydhK, aaeB, yeeA, rhtC* and *emrD,* the mutant strain being *Escherichia coli* or *Corynebacterium glutamicum.*

2. The mutant strain of claim 1, wherein the *yicL* consists of the nucleotide sequence of SEQ ID NO: 1, the *ydiN* consists of the nucleotide sequence of SEQ ID NO: 2, the *ydhK* consists of the nucleotide sequence of SEQ ID NO: 3 or 37, the *aaeB* consists of the nucleotide sequence of SEQ ID NO: 4, the *yeeA* consists of the nucleotide sequence of SEQ ID NO: 5, the *rhtC* consists of the nucleotide sequence of SEQ ID NO: 6, and the *emrD* consists of the nucleotide sequence of SEQ ID NO: 7 or 39.

3. The mutant strain of claim 1, wherein the L-amino acid is selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline.

4. A method for producing L-amino acid, the method comprising steps of:
(a) culturing the mutant strain of claim 1 in a medium; and
(b) recovering L-amino acid from the mutant strain or the medium.

5. The method of claim 4, wherein the L-amino acid is selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline.


<110>    DAESANG CORPORATION

<120>    Mutant of Escherichia coli or Mutant of Corynebacterium
         glutamicum for Producing L-amino acid and Method for Producing
         L-amino acid Using the Same

<130>    PX190055PCT

<150>    KR 10-2018-0169847
<151>    2018-12-26

<160>    39

<170>    KoPatentIn 3.0

<210>    1
<211>    924
<212>    DNA
<213>    Artificial Sequence

<220>
<223>    yicL


<400>    1
   atgggttcca ccagaaaggg gatgctgaac gttctgattg ccgccgtgtt gtggggaagt        60
   tcaggggtct gcgcgcaata catcatggag caaagccaga tgtcgtcgca gtttttgact       120
   atgacgcgtt tgatattcgc cggtttgatt ctactgacgc tgtcatttgt tcatggcgat       180
   aaaatctttt ctattattaa caatcataaa gatgccatta gcctgctgat tttttccgtg       240
   gttggcgcgc taactgtaca gctcactttt ttgctaacca tcgaaaaatc gaacgcagcc       300
   acggcaacgg tgctgcaatt cctctcaccg acgattatcg tcgcctggtt ctcactggtg       360
   cgtaaatcgc gcccgggcat tctggttttc tgcgctattt tgacatcgct ggtcgggact       420
   tttttattgg tgacacacgg taatccgacg tcattatcga tctctcctgc cgcgttgttc       480
   tggggcattg cctcggcatt tgctgctgca ttctatacca cctatccctc aacgctaatt        540
   gcccgctatg gcacgttacc agtcgtcggc tggagtatgc tgattggcgg tctgattctg       600
   ttgccttttt atgccagaca aggaacaaac tttgtcgtta acggcagttt gattctggcg       660
   ttttttattt tggtggtcat tggtacgtcc ctgacattta gtctgtacct gaaaggagca       720
   caattaattg gcggtccaaa agccagcatt ttgagctgtg cagaaccatt aagtagcgcg       780
   ctactctctt tgctgttgct ggggatcacg ttcacattac cggactggct gggaacgctg       840
   ctgattctgt catcggtgat tttgatttca atggattccc gtcgccgcgc cagaaaaata       900
   aatcgtccgg cgcggcataa gtga                                              924


<210>    2
<211>    1266
<212>    DNA
<213>    Artificial Sequence

<220>
<223>    ydiN

<400>    2
   atgtctcaaa ataaggcttt cagcacgcca tttatcctgg ctgttctttg tatttacttc      60
   agctacttcc tgcacggcat tagtgttatt acgcttgccc aaaatatgtc atctctggcg     120
   gaaaagtttt ccactgacaa cgcgggcatt gcctacttaa tttccggtat cggtttgggg     180
   cgattgatca gtattttatt cttcggtgtg atctccgata agtttggtcg tcgggcggtg     240
   atattaatgg cagtaataat gtatctgcta ttcttctttg gtattcccgc ttgcccgaat     300
   ttaactctcg cctacggtct ggcagtgtgc gtaggtatcg ctaactcagc gctggatacg     360
   ggtggctacc ccgcgctcat ggaatgcttt ccgaaagcct ctggttcggc ggtcatactg     420
   gttaaagcga tggtgtcatt tgggcaaatg ttctacccaa tgctggtgag ctatatgttg     480
   ctcaataata tctggtacgg ctatgggctg attattccgg gtattctatt tgtactgatc     540
   acgctgatgc tgttgaaaag caaattcccc agccagttgg tggacgccag cgtaactaat     600
   gaattaccgc aaatgaacag caaaccgtta gtctggctgg aaggtgtttc atcggtactg     660
   ttcggtgtag ccgcattctc gaccttttat gtgattgtgg tgtggatgcc caaatatgcg     720
   atggcttttg ctggtatgtc agaagctgag gcattaaaaa ccatctctta ttacagtatg     780
   ggctcgttgg tctgtgtctt tatttttgcc gcactactga aaaaaatggt ccggcccatc     840
   tgggctaatg tatttaactc tgcactggca acaataacag cagccattat ctacctgtac     900
   ccttctccac tggtgtgcaa tgccggagcc tttgttatcg gtttctcagc agctggcggc     960
   attttacagc tcggcgtttc ggtcatgtca gagttttttc ccaaaagcaa agccaaagtc    1020
   accagtattt atatgatgat gggtggactg gctaactttg ttattccact gattaccggt    1080
   tatctgtcga acatcggcct gcaatatatc attgttctcg attttacttt cgcgctgctg    1140
   gccctgatta ccgcaattat tgtttttatc cgctattacc gcgttttcat tattcctgaa    1200
   aatgatgtgc ggtttggcga gcgtaaattt tgcacccggt taaacacaat taagcataga    1260
   ggttaa                                                                1266

<210>    3
<211>    2013
<212>    DNA
<213>    Artificial Sequence

<220>
<223>    ydhK

<400>    3
atgaacgcat cgtcatggtc cttgcgcaat ttgccctggt tcagggccac gctggcgcaa      60

tggcgttatg cgttacgcaa taccattgcc atgtgtctgg cgctgacggt tgcctattat     120

ttaaatctgg atgaacccta ttgggcgatg acctcggctg cagtggttag ctttcccacc 180

gttggcggtg ttatcagcaa aagcctcgga cgcatcgctg gcagtttgct cggagccatt 240

gcggcactgc ttcttgccgg gcatacgctc aatgagccgt ggtttttcct attgagcatg 300

tcggcgtggc ttggcttttg tacctgggcc tgtgcgcact tcacgaataa cgtcgcgtat 360

gcatttcaac tggcgggcta cacggctgcc atcatcgcct ttccgatggt taatattact 420

gaggccagcc agctgtggga tatcgctcag gcgcgcgttt gcgaggtaat agtcggtatt 480

ttgtgcggcg gcatgatgat gatgatcctg ccgagcagtt ccgatgctac tgccctttta 540

accgcattga aaaacatgca cgcccgatta ctggaacatg ccagtttact ctggcagcct 600

gaaacaaccg atgccattcg tgcagcacat gaaggggtga ttgggcagat actgaccatg 660

aatttgctgc gtatccaggc tttctggagc cactatcgtt ttcgccagca aaacgcgcgc 720

cttaatgcgc tgctccacca gcaattacgt atgaccagtg tcatctccag cctgcgacgt 780

atgttgctca actggccctc accgccaggt gccacacgag aaattctcga acagttgctg 840

acggcgctcg ccagttcgca aacagatgtt tacaccgtcg cacgtattat cgccccgcta 900

cgcccgacca acgtcgccga ctatcggcac gtcgccttct ggcagcgact acgttatttt 960

tgccgccttt atctgcaaag tagtcaggaa ttacatcgtc tgcaaagcgg tgtagatgat 1020

cataccagac tcccacggac atccggcctg gctcgtcata ccgataacgc cgaagctatg 1080

tggagcgggc tgcgtacatt ttgtacgttg atgatgattg gcgcatggag tattgcttcg 1140

caatgggatg ccggtgccaa tgcattaacg ctggcagcaa ttagctgcgt actctactcc 1200

gccgtcgcag caccgtttaa gtcgttgtca cttctgatgc gcacgctggt gttactttcg 1260

ctattcagct ttgtggtcaa atttggtctg atggtccaga ttagcgatct gtggcaattt 1320

ttactgtttc tctttccact gctggcgaca atgcagcttc ttaaattgca gatgccaaaa 1380

tttgccgcat tgtgggggca actgattgtt tttatgggtt cttttatcgc tgtcactaat 1440

cccccggtgt atgattttgc tgattttctt aacgataatc tggcaaaaat cgttggcgtc 1500

gcgttggcgt ggttagcgtt cgccattctg cgtccaggat cggatgctcg taaaagccgc 1560

cgccatattc gcgcgctgcg ccgggatttt gtcgatcagc taagccgcca tccaacactg 1620

agtgaaagcg aatttgaatc gctcacttat catcacgtca gtcagttgag taacagccag 1680

gatgcgctgg ctcgccgttg gttattacgc tggggtgtag tgctgctgaa ctgttctcat 1740

gttgtctggc aattgcgcga ctgggaatcg cgttccgatc cgttatcgcg agtacgggat 1800

aactgtattt cactgttgcg gggagtgatg agtgagcgtg gcgttcagca aaaatcactg 1860

gcggccacac ttgaagaatt acagcggatt tgcgacagcc ttgcccgtca tcatcaacct 1920

gccgcccgtg agctggcggc aattatctgg cggctgtact gctcgctttc gcaacttgag 1980

caagcaccac cgcaaggtac gctggcctct taa 2013

<210> 4
<211> 1968
<212> DNA
<213> Artificial Sequence

<220>
<223> aaeB

<400> 4
atgggtattt tctccattgc taaccaacat attcgctttg cggtaaaact ggcgaccgcc 60

attgtactgg cgctgtttgt tggctttcac ttccagctgg aaacgccacg ctgggcggta 120

ctgacagcgg cgattgttgc cgccggtacg gcctttgctg cgggaggtga accgtattct 180

ggcgctattc gctatcgtgg ctttttgcgc atcatcggca catttattgg ctgtattgcc 240

ggactggtga tcatcattgc gatgatccgc gcaccattat tgatgattct ggtgtgctgt 300

atctgggccg gtttttgtac ctggatatcc tcgctggtac gaatagaaaa ctcgtatgcg 360

tgggggctgg ccggttatac cgcgctgatc attgtgatca ccattcagcc ggaaccattg 420

cttacgccgc agtttgccgt cgaacgttgt agcgagatcg ttatcggtat tgtgtgtgcg 480

attatggcgg atttgctctt ttctccgcga tcgatcaaac aagaagtgga tcgagagctg 540

gaaagtttgc tggtcgcgca atatcaatta atgcaactct gtatcaagca tggcgatggt 600

gaagttgtcg ataaagcctg gggcgacctg gtgcgacgca ccacggcgct acaaggcatg 660

cgcagcaacc tgaatatgga atcttcccgc tgggcgcggg ccaatcgacg tttaaaagcg 720

atcaatacgc tatcgctgac gctgattacc caatcctgcg aaacttatct tattcagaat 780

acgcgcccgg aattgatcac tgatactttc cgcgaatttt ttgacacgcc ggtagaaacc 840

gcgcaggacg tccacaagca gctcaaacgc ctgcggagag ttatcgcctg gaccggggaa 900

cgggaaacgc ctgtcaccat ttatagctgg gtcgcggcgg caacgcgtta tcagcttctc 960

aagcgcggcg ttatcagtaa cacaaaaatc aacgccaccg aagaagagat cctgcaaggc 1020

gaaccggaag taaaagtaga gtcagccgaa cgtcatcatg caatggttaa cttctggcga 1080

accacacttt cctgcattct gggcacgctt ttctggctgt ggacgggctg gacttccggc 1140

agtggtgcaa tggtgatgat tgcggtagtg acgtcactgg caatgcgttt gccgaatcca 1200

cgcatggtgg cgatcgactt tatctacggg acgctggccg cgctgccgtt agggctgctc 1260

tactttttgg tgattatccc taatacccaa cagagcatgt tgctgctgtg cattagcctg 1320

gcagtgctgg gattcttcct cggtatagaa gtacagaaac ggcgactggg ctcgatgggg 1380

gcactggcca gcaccataaa tattatcgtg ctggataacc cgatgacttt ccatttcagt 1440

cagtttctcg acagcgcatt agggcaaatc gtcggctgtg tgctcgcgtt caccgttatt 1500

ttgctggtgc gggataaatc gcgcgacagg accggacgtg tactgcttaa tcagtttgtt 1560

tctgccgctg tttccgcgat gactaccaat gtggcacgtc gtaaagagaa ccacctcccg 1620

gcactttatc agcagctgtt tttgctgatg aataagttcc caggggattt gccgaaattt 1680

cgcctggcgc tgacgatgat tatcgcgcac cagcgcctgc gtgatgcacc gatcccggtt 1740

aacgaggatt tatcggcgtt tcaccgacaa atgcgccgca cagcagacca tgtgatatct 1800

gcccgtagcg atgataaacg tcgtcggtac tttggccagt tgctggaaga actggaaatc 1860

taccaggaaa agctacgcat ctggcaagcg ccaccgcagg tgacggaacc ggtaaatcgg 1920

ctggcgggga tgctccataa gtatcaacat gcgttgaccg atagttaa 1968

<210> 5
<211> 1059
<212> DNA
<213> Artificial Sequence

<220>
<223> yeeA

<400> 5
gtgcgtgccg ataagtcatt aagcccgttt gaaatccggg tataccgcca ttaccgcatt 60

gtgcatggta ctcgggtcgc gctggcattc ctgctcactt ttctcattat ccgcctgttt 120

actatcccgg aaagcacctg gccgctggtc accatggtgg tgattatggg gccaatctcg 180

ttctggggta acgttgtccc tcgcgccttt gagcgtattg gcggtacggt gttgggttcg 240

attttaggtc ttatcgctct gcaactggag ttaatctcgt taccgctgat gttagtctgg 300

tgcgcggcgg ccatgttcct ttgcggttgg ctggcgctgg gcaagaaacc gtatcaaggt 360

ttattgattg gggtgacgct ggcaattgtt gtgggttccc cgacaggtga aattgatacg 420

gcgttatggc gaagcggcga tgtgatcctc ggctctttac tggcaatgtt gtttaccggt 480

atctggccac aacgggcgtt catccactgg cgcattcaac tggcgaaaag tctgaccgag 540

tataatcggg tctatcaatc tgcattctca ccgaacttac tcgaacgccc acgtctggaa 600

agccatctac aaaaactcct gaccgatgcc gtgaaaatgc gtggactgat tgcgcccgcc 660

agcaaagaaa cccgtattcc aaaatcgata tatgaaggta tccagaccat taaccgcaat 720

ctggtttgta tgctggagtt gcaaatcaat gcatactggg ccacgcgccc cagccatttc 780

gtgttattga acgcgcaaaa acttcgtgat acccagcaca tgatgcagca aatactgctg 840

agccttgttc atgcgctgta cgaaggtaat ccgcagccgg tttttgccaa tacggaaaaa 900

ttgaacgatg ctgtggaaga gctgcgtcag ttgctcaata accaccatga cctgaaggtt 960

gtggaaacac caatctatgg ttatgtgtgg ctgaacatgg aaacggcgca tcagcttgag 1020

ttgctatcga atctgatttg ccgggccttg cgcaaataa 1059

<210>	6
<211>	621
<212>	DNA
<213>	Artificial Sequence

<220>
<223>	rhtC


<400>	6
atgttgatgt tatttctcac cgtcgccatg gtgcacattg tggcgcttat gagccccggt	60

cccgatttct tttttgtctc tcagaccgct gtcagtcgtt cccgtaaaga agcgatgatg	120

ggcgtgctgg gcattacctg cggcgtaatg gtttgggctg ggattgcgct gcttggcctg	180

catttgatta tcgaaaaaat ggcctggctg catacgctga ttatggtggg cggtggcctg	240

tatctctgct ggatgggtta ccagatgcta cgtggtgcac tgaaaaaaga ggcggtttct	300

gcacctgcgc cacaggtcga gctggcgaaa agtgggcgca gtttcctgaa aggtttactg	360

accaatctcg ctaatccgaa agcgattatc tactttggct cggtgttctc attgtttgtc	420

ggtgataacg ttggcactac cgcgcgctgg ggcatttttg cgctgatcat tgtcgaaacg	480

ctggcgtggt ttaccgtcgt tgccagcctg tttgccctgc cgcaaatgcg ccgtggttat	540

caacgtctgg cgaagtggat tgatggtttt gccggggcgt tatttgccgg atttggcatt	600

catttgatta tttcgcggtg a	621


<210>	7
<211>	1185
<212>	DNA
<213>	Artificial Sequence

<220>
<223>	emrD


<400>	7
atgaaaaggc aaagaaacgt caatttgtta ttgatgttgg tattactcgt ggccgtcggt	60

cagatggcgc aaaccattta tattccagct attgccgata tggcgcgcga tctcaacgtc	120

cgtgaagggg cggtgcagag cgtaatgggc gcttatctgc tgacttacgg tgtctcacag	180

ctgttttatg gcccgatttc cgaccgcgtg ggccgccgac cggtgatcct cgtcggaatg	240

tccattttta tgctggcaac gctggtcgcg gtcacgacct ccagtttgac ggtgttgatt	300

gccgccagcg cgatgcaggg gatgggcacc ggcgttggcg gcgtaatggc gcgtacttta	360

ccgcgagatt tatatgaacg gacacagttg cgccatgcta acagcctgtt aaacatgggg	420

attctcgtca gtccgttgct cgcaccgcta atcggcggtc tgctggatac gatgtggaac	480

tggcgcgcct gttatctctt tttgttggtt ctttgtgctg gtgtgacctt cagtatggcc	540

cgctggatgc cggaaacgcg tccggtcgat gcaccgcgca cgcgcctgct taccagttat	600

aaaacgcttt tcggtaacag cggttttaac tgttatttgc tgatgctgat tggcggtctg 660

gccgggattg ccgcctttga agcctgctcc ggcgtgctga tgggcgcggt gttagggctg 720

agcagtatga cggtcagtat tttgtttatt ctgccgattc cggcagcgtt ttttggcgca 780

tggtttgccg gacgtcccaa taaacgcttc tccacgttaa tgtggcagtc ggttatctgc 840

tgcctgctgg ctggcttgct gatgtggatc cccgactggt ttggcgtgat gaatgtctgg 900

acgctgctcg ttcccgccgc gctgttcttt ttcggtgccg ggatgctgtt tccgctggcg 960

accagcggcg cgatggagcc gttccccttc ctggcgggca cggctggcgc gctggtcggc 1020

ggtctgcaaa acattggttc cggcgtgctg gcgtcgctct ctgcgatgtt accgcaaacc 1080

ggtcagggca gcctggggtt gttgatgacc ttaatgggat tgttgatcgt gctgtgctgg 1140

ctgccgctgg cgacgcggat gtcgcatcag gggcagcccg tttaa 1185

<210> 8
<211> 307
<212> PRT
<213> Artificial Sequence

<220>
<223> yicL

<400> 8
   Met Gly Ser Thr Arg Lys Gly Met Leu Asn Val Leu Ile Ala Ala Val
   1 5 10 15
   Leu Trp Gly Ser Ser Gly Val Cys Ala Gln Tyr Ile Met Glu Gln Ser
   20 25 30
   Gln Met Ser Ser Gln Phe Leu Thr Met Thr Arg Leu Ile Phe Ala Gly
   35 40 45
   Leu Ile Leu Leu Thr Leu Ser Phe Val His Gly Asp Lys Ile Phe Ser
   50 55 60
   Ile Ile Asn Asn His Lys Asp Ala Ile Ser Leu Leu Ile Phe Ser Val
   65 70 75 80
   Val Gly Ala Leu Thr Val Gln Leu Thr Phe Leu Leu Thr Ile Glu Lys
   85 90 95
   Ser Asn Ala Ala Thr Ala Thr Val Leu Gln Phe Leu Ser Pro Thr Ile
   100 105 110
   Ile Val Ala Trp Phe Ser Leu Val Arg Lys Ser Arg Pro Gly Ile Leu
   115 120 125
   Val Phe Cys Ala Ile Leu Thr Ser Leu Val Gly Thr Phe Leu Leu Val
   130 135 140
   Thr His Gly Asn Pro Thr Ser Leu Ser Ile Ser Pro Ala Ala Leu Phe
   145 150 155 160
   Trp Gly Ile Ala Ser Ala Phe Ala Ala Ala Phe Tyr Thr Thr Tyr Pro
   165 170 175
   Ser Thr Leu Ile Ala Arg Tyr Gly Thr Leu Pro Val Val Gly Trp Ser
   180 185 190
   Met Leu Ile Gly Gly Leu Ile Leu Leu Pro Phe Tyr Ala Arg Gln Gly
   195 200 205
   Thr Asn Phe Val Val Asn Gly Ser Leu Ile Leu Ala Phe Phe Tyr Leu
   210 215 220
   Val Val Ile Gly Thr Ser Leu Thr Phe Ser Leu Tyr Leu Lys Gly Ala
   225 230 235 240
   Gln Leu Ile Gly Gly Pro Lys Ala Ser Ile Leu Ser Cys Ala Glu Pro
   245 250 255
   Leu Ser Ser Ala Leu Leu Ser Leu Leu Leu Leu Gly Ile Thr Phe Thr
   260 265 270
   Leu Pro Asp Trp Leu Gly Thr Leu Leu Ile Leu Ser Ser Val Ile Leu
   275 280 285
   Ile Ser Met Asp Ser Arg Arg Arg Ala Arg Lys Ile Asn Arg Pro Ala
   290 295 300
   Arg His Lys
   305

<210> 9
<211> 421
<212> PRT
<213> Artificial Sequence

<220>
<223> ydiN

<400> 9
Met Ser Gln Asn Lys Ala Phe Ser Thr Pro Phe Ile Leu Ala Val Leu
1               5                   10                  15

Cys Ile Tyr Phe Ser Tyr Phe Leu His Gly Ile Ser Val Ile Thr Leu
            20                  25                  30

Ala Gln Asn Met Ser Ser Leu Ala Glu Lys Phe Ser Thr Asp Asn Ala
        35                  40                  45

Gly Ile Ala Tyr Leu Ile Ser Gly Ile Gly Leu Gly Arg Leu Ile Ser
    50                  55                  60

Ile Leu Phe Phe Gly Val Ile Ser Asp Lys Phe Gly Arg Arg Ala Val
65                  70                  75                  80

Ile Leu Met Ala Val Ile Met Tyr Leu Leu Phe Phe Phe Gly Ile Pro
                85                  90                  95

Ala Cys Pro Asn Leu Thr Leu Ala Tyr Gly Leu Ala Val Cys Val Gly
            100                 105                 110

Ile Ala Asn Ser Ala Leu Asp Thr Gly Gly Tyr Pro Ala Leu Met Glu
        115                 120                 125

Cys Phe Pro Lys Ala Ser Gly Ser Ala Val Ile Leu Val Lys Ala Met
    130                 135                 140

Val Ser Phe Gly Gln Met Phe Tyr Pro Met Leu Val Ser Tyr Met Leu
145                 150                 155                 160

Leu Asn Asn Ile Trp Tyr Gly Tyr Gly Leu Ile Ile Pro Gly Ile Leu
                165                 170                 175

Phe Val Leu Ile Thr Leu Met Leu Leu Lys Ser Lys Phe Pro Ser Gln
            180                 185                 190

Leu Val Asp Ala Ser Val Thr Asn Glu Leu Pro Gln Met Asn Ser Lys
        195                 200                 205

Pro Leu Val Trp Leu Glu Gly Val Ser Ser Val Leu Phe Gly Val Ala
    210                 215                 220

Ala Phe Ser Thr Phe Tyr Val Ile Val Val Trp Met Pro Lys Tyr Ala
225                 230                 235                 240

Met Ala Phe Ala Gly Met Ser Glu Ala Glu Ala Leu Lys Thr Ile Ser
                245                 250                 255

Tyr Tyr Ser Met Gly Ser Leu Val Cys Val Phe Ile Phe Ala Ala Leu
            260                 265                 270

Leu Lys Lys Met Val Arg Pro Ile Trp Ala Asn Val Phe Asn Ser Ala
        275                 280                 285

Leu Ala Thr Ile Thr Ala Ala Ile Ile Tyr Leu Tyr Pro Ser Pro Leu
    290                 295                 300

Val Cys Asn Ala Gly Ala Phe Val Ile Gly Phe Ser Ala Ala Gly Gly
305                 310                 315                 320

Ile Leu Gln Leu Gly Val Ser Val Met Ser Glu Phe Phe Pro Lys Ser
                325                 330                 335

Lys Ala Lys Val Thr Ser Ile Tyr Met Met Met Gly Gly Leu Ala Asn
            340                 345                 350

Phe Val Ile Pro Leu Ile Thr Gly Tyr Leu Ser Asn Ile Gly Leu Gln
        355                 360                 365

Tyr Ile Ile Val Leu Asp Phe Thr Phe Ala Leu Leu Ala Leu Ile Thr
    370                 375                 380

Ala Ile Ile Val Phe Ile Arg Tyr Tyr Arg Val Phe Ile Ile Pro Glu
385                 390                 395                 400

Asn Asp Val Arg Phe Gly Glu Arg Lys Phe Cys Thr Arg Leu Asn Thr
                405                 410                 415

Ile Lys His Arg Gly
            420


<210>    10
<211>    670
<212>    PRT
<213>    Artificial Sequence

<220>
<223>    ydhK

<400>    10
Met Asn Ala Ser Ser Trp Ser Leu Arg Asn Leu Pro Trp Phe Arg Ala
1               5                   10                  15

Thr Leu Ala Gln Trp Arg Tyr Ala Leu Arg Asn Thr Ile Ala Met Cys
            20                  25                  30

Leu Ala Leu Thr Val Ala Tyr Tyr Leu Asn Leu Asp Glu Pro Tyr Trp
        35                  40                  45

Ala Met Thr Ser Ala Ala Val Val Ser Phe Pro Thr Val Gly Gly Val
    50                  55                  60

Ile Ser Lys Ser Leu Gly Arg Ile Ala Gly Ser Leu Leu Gly Ala Ile
65                  70                  75                  80

Ala Ala Leu Leu Leu Ala Gly His Thr Leu Asn Glu Pro Trp Phe Phe
                85                  90                  95

Leu Leu Ser Met Ser Ala Trp Leu Gly Phe Cys Thr Trp Ala Cys Ala
            100                 105                 110

His Phe Thr Asn Asn Val Ala Tyr Ala Phe Gln Leu Ala Gly Tyr Thr
        115                 120                 125

Ala Ala Ile Ile Ala Phe Pro Met Val Asn Ile Thr Glu Ala Ser Gln
    130                 135                 140

Leu Trp Asp Ile Ala Gln Ala Arg Val Cys Glu Val Ile Val Gly Ile
145                 150                 155                 160

Leu Cys Gly Gly Met Met Met Met Ile Leu Pro Ser Ser Ser Asp Ala
                165                 170                 175

Thr Ala Leu Leu Thr Ala Leu Lys Asn Met His Ala Arg Leu Leu Glu
            180                 185                 190

His Ala Ser Leu Leu Trp Gln Pro Glu Thr Thr Asp Ala Ile Arg Ala
        195                 200                 205

Ala His Glu Gly Val Ile Gly Gln Ile Leu Thr Met Asn Leu Leu Arg
    210                 215                 220

Ile Gln Ala Phe Trp Ser His Tyr Arg Phe Arg Gln Gln Asn Ala Arg
225                 230                 235                 240

Leu Asn Ala Leu Leu His Gln Gln Leu Arg Met Thr Ser Val Ile Ser
                245                 250                 255

Ser Leu Arg Arg Met Leu Leu Asn Trp Pro Ser Pro Pro Gly Ala Thr
            260                 265                 270

Arg Glu Ile Leu Glu Gln Leu Leu Thr Ala Leu Ala Ser Ser Gln Thr
        275                 280                 285

Asp Val Tyr Thr Val Ala Arg Ile Ile Ala Pro Leu Arg Pro Thr Asn
    290                 295                 300

Val Ala Asp Tyr Arg His Val Ala Phe Trp Gln Arg Leu Arg Tyr Phe
305                 310                 315                 320

Cys Arg Leu Tyr Leu Gln Ser Ser Gln Glu Leu His Arg Leu Gln Ser
                325                 330                 335

Gly Val Asp Asp His Thr Arg Leu Pro Arg Thr Ser Gly Leu Ala Arg
            340                 345                 350

His Thr Asp Asn Ala Glu Ala Met Trp Ser Gly Leu Arg Thr Phe Cys
        355                 360                 365

Thr Leu Met Met Ile Gly Ala Trp Ser Ile Ala Ser Gln Trp Asp Ala
    370                 375                 380

Gly Ala Asn Ala Leu Thr Leu Ala Ala Ile Ser Cys Val Leu Tyr Ser
385                 390                 395                 400

Ala Val Ala Ala Pro Phe Lys Ser Leu Ser Leu Leu Met Arg Thr Leu
                405                 410                 415

Val Leu Leu Ser Leu Phe Ser Phe Val Val Lys Phe Gly Leu Met Val
            420                 425                 430

Gln Ile Ser Asp Leu Trp Gln Phe Leu Leu Phe Leu Phe Pro Leu Leu
        435                 440                 445

Ala Thr Met Gln Leu Leu Lys Leu Gln Met Pro Lys Phe Ala Ala Leu
    450                 455                 460

Trp Gly Gln Leu Ile Val Phe Met Gly Ser Phe Ile Ala Val Thr Asn
465                 470                 475                 480

Pro Pro Val Tyr Asp Phe Ala Asp Phe Leu Asn Asp Asn Leu Ala Lys
                485                 490                 495

Ile Val Gly Val Ala Leu Ala Trp Leu Ala Phe Ala Ile Leu Arg Pro
            500                 505                 510

Gly Ser Asp Ala Arg Lys Ser Arg Arg His Ile Arg Ala Leu Arg Arg
        515                 520                 525

Asp Phe Val Asp Gln Leu Ser Arg His Pro Thr Leu Ser Glu Ser Glu
    530                 535                 540

Phe Glu Ser Leu Thr Tyr His His Val Ser Gln Leu Ser Asn Ser Gln
545                 550                 555                 560

Asp Ala Leu Ala Arg Arg Trp Leu Leu Arg Trp Gly Val Val Leu Leu
                565                 570                 575

Asn Cys Ser His Val Val Trp Gln Leu Arg Asp Trp Glu Ser Arg Ser
            580                 585                 590

Asp Pro Leu Ser Arg Val Arg Asp Asn Cys Ile Ser Leu Leu Arg Gly
        595                 600                 605

Val Met Ser Glu Arg Gly Val Gln Gln Lys Ser Leu Ala Ala Thr Leu
    610                 615                 620

Glu Glu Leu Gln Arg Ile Cys Asp Ser Leu Ala Arg His His Gln Pro
625                 630                 635                 640

Ala Ala Arg Glu Leu Ala Ala Ile Ile Trp Arg Leu Tyr Cys Ser Leu
                645                 650                 655

Ser Gln Leu Glu Gln Ala Pro Pro Gln Gly Thr Leu Ala Ser
            660                 665                 670

<210>  11
<211>  655
<212>  PRT
<213>  Artificial Sequence

<220>
<223>  aaeB

<400>  11
Met Gly Ile Phe Ser Ile Ala Asn Gln His Ile Arg Phe Ala Val Lys
1               5                   10                  15

Leu Ala Thr Ala Ile Val Leu Ala Leu Phe Val Gly Phe His Phe Gln
            20                  25                  30

Leu Glu Thr Pro Arg Trp Ala Val Leu Thr Ala Ala Ile Val Ala Ala
        35                  40                  45

Gly Thr Ala Phe Ala Ala Gly Gly Glu Pro Tyr Ser Gly Ala Ile Arg
    50                  55                  60

Tyr Arg Gly Phe Leu Arg Ile Ile Gly Thr Phe Ile Gly Cys Ile Ala
65                  70                  75                  80

Gly Leu Val Ile Ile Ile Ala Met Ile Arg Ala Pro Leu Leu Met Ile
                85                  90                  95

Leu Val Cys Cys Ile Trp Ala Gly Phe Cys Thr Trp Ile Ser Ser Leu
            100                 105                 110

Val Arg Ile Glu Asn Ser Tyr Ala Trp Gly Leu Ala Gly Tyr Thr Ala
        115                 120                 125

Leu Ile Ile Val Ile Thr Ile Gln Pro Glu Pro Leu Leu Thr Pro Gln
    130                 135                 140

Phe Ala Val Glu Arg Cys Ser Glu Ile Val Ile Gly Ile Val Cys Ala
145                 150                 155                 160

Ile Met Ala Asp Leu Leu Phe Ser Pro Arg Ser Ile Lys Gln Glu Val
                165                 170                 175

Asp Arg Glu Leu Glu Ser Leu Leu Val Ala Gln Tyr Gln Leu Met Gln
            180                 185                 190

Leu Cys Ile Lys His Gly Asp Gly Glu Val Val Asp Lys Ala Trp Gly
        195                 200                 205

Asp Leu Val Arg Arg Thr Thr Ala Leu Gln Gly Met Arg Ser Asn Leu
    210                 215                 220

Asn Met Glu Ser Ser Arg Trp Ala Arg Ala Asn Arg Arg Leu Lys Ala
225                 230                 235                 240

Ile Asn Thr Leu Ser Leu Thr Leu Ile Thr Gln Ser Cys Glu Thr Tyr

245                 250                 255

Leu Ile Gln Asn Thr Arg Pro Glu Leu Ile Thr Asp Thr Phe Arg Glu
            260                 265                 270

Phe Phe Asp Thr Pro Val Glu Thr Ala Gln Asp Val His Lys Gln Leu
        275                 280                 285

Lys Arg Leu Arg Arg Val Ile Ala Trp Thr Gly Glu Arg Glu Thr Pro
    290                 295                 300

Val Thr Ile Tyr Ser Trp Val Ala Ala Ala Thr Arg Tyr Gln Leu Leu
305                 310                 315                 320

Lys Arg Gly Val Ile Ser Asn Thr Lys Ile Asn Ala Thr Glu Glu Glu
                325                 330                 335

Ile Leu Gln Gly Glu Pro Glu Val Lys Val Glu Ser Ala Glu Arg His
            340                 345                 350

His Ala Met Val Asn Phe Trp Arg Thr Thr Leu Ser Cys Ile Leu Gly
        355                 360                 365

Thr Leu Phe Trp Leu Trp Thr Gly Trp Thr Ser Gly Ser Gly Ala Met
    370                 375                 380

Val Met Ile Ala Val Val Thr Ser Leu Ala Met Arg Leu Pro Asn Pro
385                 390                 395                 400

Arg Met Val Ala Ile Asp Phe Ile Tyr Gly Thr Leu Ala Ala Leu Pro
                405                 410                 415

Leu Gly Leu Leu Tyr Phe Leu Val Ile Ile Pro Asn Thr Gln Gln Ser
            420                 425                 430

Met Leu Leu Leu Cys Ile Ser Leu Ala Val Leu Gly Phe Phe Leu Gly
        435                 440                 445

Ile Glu Val Gln Lys Arg Arg Leu Gly Ser Met Gly Ala Leu Ala Ser
    450                 455                 460

Thr Ile Asn Ile Ile Val Leu Asp Asn Pro Met Thr Phe His Phe Ser
465                 470                 475                 480

Gln Phe Leu Asp Ser Ala Leu Gly Gln Ile Val Gly Cys Val Leu Ala
                485                 490                 495

Phe Thr Val Ile Leu Leu Val Arg Asp Lys Ser Arg Asp Arg Thr Gly
            500                 505                 510

Arg Val Leu Leu Asn Gln Phe Val Ser Ala Ala Val Ser Ala Met Thr
        515                 520                 525

Thr Asn Val Ala Arg Arg Lys Glu Asn His Leu Pro Ala Leu Tyr Gln
    530                 535                 540

Gln Leu Phe Leu Leu Met Asn Lys Phe Pro Gly Asp Leu Pro Lys Phe
545                 550                 555                 560

Arg Leu Ala Leu Thr Met Ile Ile Ala His Gln Arg Leu Arg Asp Ala
                565                 570                 575

Pro Ile Pro Val Asn Glu Asp Leu Ser Ala Phe His Arg Gln Met Arg

580                 585                 590

Arg Thr Ala Asp His Val Ile Ser Ala Arg Ser Asp Asp Lys Arg Arg
        595                 600                 605

Arg Tyr Phe Gly Gln Leu Leu Glu Glu Leu Glu Ile Tyr Gln Glu Lys
    610                 615                 620

Leu Arg Ile Trp Gln Ala Pro Pro Gln Val Thr Glu Pro Val Asn Arg
625                 630                 635                 640

Leu Ala Gly Met Leu His Lys Tyr Gln His Ala Leu Thr Asp Ser
                645                 650                 655

<210>    12
<211>    352
<212>    PRT
<213>    Artificial Sequence

<220>
<223>    yeeA

<400>    12
Val Arg Ala Asp Lys Ser Leu Ser Pro Phe Glu Ile Arg Val Tyr Arg
 1               5                  10                  15

His Tyr Arg Ile Val His Gly Thr Arg Val Ala Leu Ala Phe Leu Leu
            20                  25                  30

Thr Phe Leu Ile Ile Arg Leu Phe Thr Ile Pro Glu Ser Thr Trp Pro
        35                  40                  45

Leu Val Thr Met Val Val Ile Met Gly Pro Ile Ser Phe Trp Gly Asn
    50                  55                  60

Val Val Pro Arg Ala Phe Glu Arg Ile Gly Gly Thr Val Leu Gly Ser
65                  70                  75                  80

Ile Leu Gly Leu Ile Ala Leu Gln Leu Glu Leu Ile Ser Leu Pro Leu
                85                  90                  95

Met Leu Val Trp Cys Ala Ala Ala Met Phe Leu Cys Gly Trp Leu Ala
            100                 105                 110

Leu Gly Lys Lys Pro Tyr Gln Gly Leu Leu Ile Gly Val Thr Leu Ala
        115                 120                 125

Ile Val Val Gly Ser Pro Thr Gly Glu Ile Asp Thr Ala Leu Trp Arg
    130                 135                 140

Ser Gly Asp Val Ile Leu Gly Ser Leu Leu Ala Met Leu Phe Thr Gly
145                 150                 155                 160

Ile Trp Pro Gln Arg Ala Phe Ile His Trp Arg Ile Gln Leu Ala Lys
                165                 170                 175

Ser Leu Thr Glu Tyr Asn Arg Val Tyr Gln Ser Ala Phe Ser Pro Asn
            180                 185                 190

Leu Leu Glu Arg Pro Arg Leu Glu Ser His Leu Gln Lys Leu Leu Thr
        195                 200                 205

Asp Ala Val Lys Met Arg Gly Leu Ile Ala Pro Ala Ser Lys Glu Thr
    210                 215                 220

Arg Ile Pro Lys Ser Ile Tyr Glu Gly Ile Gln Thr Ile Asn Arg Asn
225                 230                 235                 240

Leu Val Cys Met Leu Glu Leu Gln Ile Asn Ala Tyr Trp Ala Thr Arg
                245                 250                 255

Pro Ser His Phe Val Leu Leu Asn Ala Gln Lys Leu Arg Asp Thr Gln
            260                 265                 270

His Met Met Gln Gln Ile Leu Leu Ser Leu Val His Ala Leu Tyr Glu
        275                 280                 285

Gly Asn Pro Gln Pro Val Phe Ala Asn Thr Glu Lys Leu Asn Asp Ala
    290                 295                 300

Val Glu Glu Leu Arg Gln Leu Leu Asn Asn His His Asp Leu Lys Val
305                 310                 315                 320

Val Glu Thr Pro Ile Tyr Gly Tyr Val Trp Leu Asn Met Glu Thr Ala
                325                 330                 335

His Gln Leu Glu Leu Leu Ser Asn Leu Ile Cys Arg Ala Leu Arg Lys
            340                 345                 350

<210>    13
<211>    206
<212>    PRT
<213>    Artificial Sequence

<220>
<223>    rhtC

<400>    13
Met Leu Met Leu Phe Leu Thr Val Ala Met Val His Ile Val Ala Leu
 1               5                  10                  15

Met Ser Pro Gly Pro Asp Phe Phe Phe Val Ser Gln Thr Ala Val Ser
            20                  25                  30

Arg Ser Arg Lys Glu Ala Met Met Gly Val Leu Gly Ile Thr Cys Gly
        35                  40                  45

Val Met Val Trp Ala Gly Ile Ala Leu Leu Gly Leu His Leu Ile Ile
    50                  55                  60

Glu Lys Met Ala Trp Leu His Thr Leu Ile Met Val Gly Gly Gly Leu
 65                 70                  75                  80

Tyr Leu Cys Trp Met Gly Tyr Gln Met Leu Arg Gly Ala Leu Lys Lys
                85                  90                  95

Glu Ala Val Ser Ala Pro Ala Pro Gln Val Glu Leu Ala Lys Ser Gly
            100                 105                 110

Arg Ser Phe Leu Lys Gly Leu Leu Thr Asn Leu Ala Asn Pro Lys Ala

115                 120                 125

Ile Ile Tyr Phe Gly Ser Val Phe Ser Leu Phe Val Gly Asp Asn Val
    130                 135                 140

Gly Thr Thr Ala Arg Trp Gly Ile Phe Ala Leu Ile Ile Val Glu Thr
145                 150                 155                 160

Leu Ala Trp Phe Thr Val Val Ala Ser Leu Phe Ala Leu Pro Gln Met
                165                 170                 175

Arg Arg Gly Tyr Gln Arg Leu Ala Lys Trp Ile Asp Gly Phe Ala Gly
            180                 185                 190

Ala Leu Phe Ala Gly Phe Gly Ile His Leu Ile Ile Ser Arg
        195                 200                 205

<210>    14
<211>    394
<212>    PRT
<213>    Artificial Sequence

<220>
<223>    emrD

<400>    14
Met Lys Arg Gln Arg Asn Val Asn Leu Leu Leu Met Leu Val Leu Leu
1               5                   10                  15

Val Ala Val Gly Gln Met Ala Gln Thr Ile Tyr Ile Pro Ala Ile Ala
            20                  25                  30

Asp Met Ala Arg Asp Leu Asn Val Arg Glu Gly Ala Val Gln Ser Val
        35                  40                  45

Met Gly Ala Tyr Leu Leu Thr Tyr Gly Val Ser Gln Leu Phe Tyr Gly
    50                  55                  60

Pro Ile Ser Asp Arg Val Gly Arg Arg Pro Val Ile Leu Val Gly Met
65                  70                  75                  80

Ser Ile Phe Met Leu Ala Thr Leu Val Ala Val Thr Thr Ser Ser Leu
                85                  90                  95

Thr Val Leu Ile Ala Ala Ser Ala Met Gln Gly Met Gly Thr Gly Val
            100                 105                 110

Gly Gly Val Met Ala Arg Thr Leu Pro Arg Asp Leu Tyr Glu Arg Thr
        115                 120                 125

Gln Leu Arg His Ala Asn Ser Leu Leu Asn Met Gly Ile Leu Val Ser
    130                 135                 140

Pro Leu Leu Ala Pro Leu Ile Gly Gly Leu Leu Asp Thr Met Trp Asn
145                 150                 155                 160

Trp Arg Ala Cys Tyr Leu Phe Leu Leu Val Leu Cys Ala Gly Val Thr
                165                 170                 175

Phe Ser Met Ala Arg Trp Met Pro Glu Thr Arg Pro Val Asp Ala Pro

180                 185                 190

Arg Thr Arg Leu Leu Thr Ser Tyr Lys Thr Leu Phe Gly Asn Ser Gly
        195                 200                 205

Phe Asn Cys Tyr Leu Leu Met Leu Ile Gly Gly Leu Ala Gly Ile Ala
    210                 215                 220

Ala Phe Glu Ala Cys Ser Gly Val Leu Met Gly Ala Val Leu Gly Leu
225                 230                 235                 240

Ser Ser Met Thr Val Ser Ile Leu Phe Ile Leu Pro Ile Pro Ala Ala
                245                 250                 255

Phe Phe Gly Ala Trp Phe Ala Gly Arg Pro Asn Lys Arg Phe Ser Thr
            260                 265                 270

Leu Met Trp Gln Ser Val Ile Cys Cys Leu Leu Ala Gly Leu Leu Met
        275                 280                 285

Trp Ile Pro Asp Trp Phe Gly Val Met Asn Val Trp Thr Leu Leu Val
    290                 295                 300

Pro Ala Ala Leu Phe Phe Phe Gly Ala Gly Met Leu Phe Pro Leu Ala
305                 310                 315                 320

Thr Ser Gly Ala Met Glu Pro Phe Pro Phe Leu Ala Gly Thr Ala Gly
                325                 330                 335

Ala Leu Val Gly Gly Leu Gln Asn Ile Gly Ser Gly Val Leu Ala Ser
            340                 345                 350

Leu Ser Ala Met Leu Pro Gln Thr Gly Gln Gly Ser Leu Gly Leu Leu
        355                 360                 365

Met Thr Leu Met Gly Leu Leu Ile Val Leu Cys Trp Leu Pro Leu Ala
    370                 375                 380

Thr Arg Met Ser His Gln Gly Gln Pro Val
385                 390


<210>    15
<211>    25
<212>    DNA
<213>    Artificial Sequence

<220>
<223>    yddG H1F


<400>    15
caatgccgct actgttgttc cagcc                                          25


<210>    16
<211>    25
<212>    DNA
<213>    Artificial Sequence

<220>
<223>    yddG H1R

<400>    16
cagtggtgcg tttttctacc gctat                                          25


<210>    17
<211>    22
<212>    DNA
<213>    Artificial Sequence

<220>
<223>    yddG H2F


<400>    17
aataactgcc gggtctacgg cc                                             22


<210>    18
<211>    33
<212>    DNA
<213>    Artificial Sequence

<220>
<223>    yddG H2R


<400>    18
aacgtatttt ctaaacgaat tttaaacggc gtc                                 33


<210>    19
<211>    26
<212>    DNA
<213>    Artificial Sequence

<220>
<223>    yddG CF


<400>    19
cggaacagta tgtgcaggtg ttacgg                                         26


<210>    20
<211>    26
<212>    DNA
<213>    Artificial Sequence

<220>
<223>    yddG CR


<400>    20
aacaaaccag ttacaaccac cgcaac                                         26


<210>    21
<211>    28
<212>    DNA
<213>    Artificial Sequence

<220>

<223>    yicL_F

<400>    21
gcgagctcat gggttccacc agaaaggg                                          28

<210>    22
<211>    27
<212>    DNA
<213>    Artificial Sequence

<220>
<223>    yicL_R

<400>    22
gctctagatc acttatgccg cgccgga                                           27

<210>    23
<211>    33
<212>    DNA
<213>    Artificial Sequence

<220>
<223>    ydiN_F

<400>    23
gcgagctcat gtctcaaaat aaggctttca gca                                    33

<210>    24
<211>    28
<212>    DNA
<213>    Artificial Sequence

<220>
<223>    ydiN_R

<400>    24
gctctagagg ccatcaaccc aatcaatt                                          28

<210>    25
<211>    33
<212>    DNA
<213>    Artificial Sequence

<220>
<223>    ydhK_F

<400>    25
gcgagctcat gaacgcatcg tcatggtcct tgc                                    33

<210>    26
<211>    27
<212>    DNA
<213>    Artificial Sequence

<220>
<223>    ydhK_R

<400>    26
gctctagatc acttatgccg cgccgga                                          27

<210>    27
<211>    29
<212>    DNA
<213>    Artificial Sequence

<220>
<223>    aaeB_F

<400>    27
gcgagctcat gggtattttc tccattgct                                        29

<210>    28
<211>    29
<212>    DNA
<213>    Artificial Sequence

<220>
<223>    aaeB_R

<400>    28
gctctagatt ttgacttaac tatcggtca                                        29

<210>    29
<211>    25
<212>    DNA
<213>    Artificial Sequence

<220>
<223>    yeeA_F

<400>    29
gcgagctcgt gcgtgccgat aagtc                                            25

<210>    30
<211>    26
<212>    DNA
<213>    Artificial Sequence

<220>
<223>    yeeA_R

<400>    30
gctctagatt atttgcgcaa ggcccg                                           26

<210>    31
<211>    31

<212>    DNA
<213>    Artificial Sequence

<220>
<223>    rhtC_F


<400>    31
gcgagctcat gttgatgtta tttctcaccg t                                    31


<210>    32
<211>    28
<212>    DNA
<213>    Artificial Sequence

<220>
<223>    rhtC_R


<400>    32
gctctagact ggcatcaccg cgaaataa                                        28


<210>    33
<211>    27
<212>    DNA
<213>    Artificial Sequence

<220>
<223>    emrD_F


<400>    33
gcgagctcat gaaaaggcaa agaaacg                                         27


<210>    34
<211>    27
<212>    DNA
<213>    Artificial Sequence

<220>
<223>    emrD_R


<400>    34
gctctagacg gtgacgtgcg cttaaac                                         27


<210>    35
<211>    24
<212>    DNA
<213>    Artificial Sequence

<220>
<223>    primer_F


<400>    35
gcgccgacat cataacggtt ctgg                                            24

<210>    36
<211>    23
<212>    DNA
<213>    Artificial Sequence

<220>
<223>    primer_R


<400>    36
cgcaacgttc aaatccgctc ccg                                              23


<210>    37
<211>    2013
<212>    DNA
<213>    Artificial Sequence

<220>
<223>    ydhK 1945G


<400>    37
atgaacgcat cgtcatggtc cttgcgcaat ttgccctggt tcagggccac gctggcgcaa      60

tggcgttatg cgttacgcaa taccattgcc atgtgtctgg cgctgacggt tgcctattat     120

ttaaatctgg atgaacccta ttgggcgatg acctcggctg cagtggttag ctttcccacc     180

gttggcggtg ttatcagcaa aagcctcgga cgcatcgctg gcagtttgct cggagccatt     240

gcggcactgc ttcttgccgg gcatacgctc aatgagccgt ggtttttcct attgagcatg     300

tcggcgtggc ttggcttttg tacctgggcc tgtgcgcact tcacgaataa cgtcgcgtat     360

gcatttcaac tggcgggcta cacggctgcc atcatcgcct ttccgatggt taatattact     420

gaggccagcc agctgtggga tatcgctcag gcgcgcgttt gcgaggtaat agtcggtatt     480

ttgtgcggcg gcatgatgat gatgatcctg ccgagcagtt ccgatgctac tgccctttta     540

accgcattga aaaacatgca cgcccgatta ctggaacatg ccagtttact ctggcagcct     600

gaaacaaccg atgccattcg tgcagcacat gaaggggtga ttgggcagat actgaccatg     660

aatttgctgc gtatccaggc tttctggagc cactatcgtt ttcgccagca aaacgcgcgc     720

cttaatgcgc tgctccacca gcaattacgt atgaccagtg tcatctccag cctgcgacgt     780

atgttgctca actggccctc accgccaggt gccacacgag aaattctcga acagttgctg     840

acggcgctcg ccagttcgca aacagatgtt tacaccgtcg cacgtattat cgccccgcta     900

cgcccgacca acgtcgccga ctatcggcac gtcgccttct ggcagcgact acgttatttt     960

tgccgccttt atctgcaaag tagtcaggaa ttacatcgtc tgcaaagcgg tgtagatgat    1020

cataccagac tcccacggac atccggcctg gctcgtcata ccgataacgc cgaagctatg    1080

tggagcgggc tgcgtacatt ttgtacgttg atgatgattg gcgcatggag tattgcttcg    1140

caatgggatg ccggtgccaa tgcattaacg ctggcagcaa ttagctgcgt actctactcc    1200

gccgtcgcag caccgtttaa gtcgttgtca cttctgatgc gcacgctggt gttactttcg 1260

ctattcagct ttgtggtcaa atttggtctg atggtccaga ttagcgatct gtggcaattt 1320

ttactgtttc tctttccact gctggcgaca atgcagcttc ttaaattgca gatgccaaaa 1380

tttgccgcat tgtgggggca actgattgtt tttatgggtt cttttatcgc tgtcactaat 1440

cccccggtgt atgattttgc tgattttctt aacgataatc tggcaaaaat cgttggcgtc 1500

gcgttggcgt ggttagcgtt cgccattctg cgtccaggat cggatgctcg taaaagccgc 1560

cgccatattc gcgcgctgcg ccgggatttt gtcgatcagc taagccgcca tccaacactg 1620

agtgaaagcg aatttgaatc gctcacttat catcacgtca gtcagttgag taacagccag 1680

gatgcgctgg ctcgccgttg gttattacgc tggggtgtag tgctgctgaa ctgttctcat 1740

gttgtctggc aattgcgcga ctgggaatcg cgttccgatc cgttatcgcg agtacgggat 1800

aactgtattt cactgttgcg gggagtgatg agtgagcgtg gcgttcagca aaaatcactg 1860

gcggccacac ttgaagaatt acagcggatt tgcgacagcc ttgcccgtca tcatcaacct 1920

gccgcccgtg agctggcggc aattgtctgg cggctgtact gctcgctttc gcaacttgag 1980

caagcaccac cgcaaggtac gctggcctct taa 2013

<210> 38
<211> 670
<212> PRT
<213> Artificial Sequence

<220>
<223> ydhK 649V

<400> 38
Met Asn Ala Ser Ser Trp Ser Leu Arg Asn Leu Pro Trp Phe Arg Ala
1               5                   10                  15

Thr Leu Ala Gln Trp Arg Tyr Ala Leu Arg Asn Thr Ile Ala Met Cys
            20                  25                  30

Leu Ala Leu Thr Val Ala Tyr Tyr Leu Asn Leu Asp Glu Pro Tyr Trp
        35                  40                  45

Ala Met Thr Ser Ala Ala Val Val Ser Phe Pro Thr Val Gly Gly Val
    50                  55                  60

Ile Ser Lys Ser Leu Gly Arg Ile Ala Gly Ser Leu Leu Gly Ala Ile
65                  70                  75                  80

Ala Ala Leu Leu Leu Ala Gly His Thr Leu Asn Glu Pro Trp Phe Phe
                85                  90                  95

Leu Leu Ser Met Ser Ala Trp Leu Gly Phe Cys Thr Trp Ala Cys Ala
            100                 105                 110

His Phe Thr Asn Asn Val Ala Tyr Ala Phe Gln Leu Ala Gly Tyr Thr
        115                 120                 125

Ala Ala Ile Ile Ala Phe Pro Met Val Asn Ile Thr Glu Ala Ser Gln
    130                 135                 140

Leu Trp Asp Ile Ala Gln Ala Arg Val Cys Glu Val Ile Val Gly Ile
145                 150                 155                 160

Leu Cys Gly Gly Met Met Met Met Ile Leu Pro Ser Ser Ser Asp Ala
                165                 170                 175

Thr Ala Leu Leu Thr Ala Leu Lys Asn Met His Ala Arg Leu Leu Glu
            180                 185                 190

His Ala Ser Leu Leu Trp Gln Pro Glu Thr Thr Asp Ala Ile Arg Ala
        195                 200                 205

Ala His Glu Gly Val Ile Gly Gln Ile Leu Thr Met Asn Leu Leu Arg
    210                 215                 220

Ile Gln Ala Phe Trp Ser His Tyr Arg Phe Arg Gln Gln Asn Ala Arg
225                 230                 235                 240

Leu Asn Ala Leu Leu His Gln Gln Leu Arg Met Thr Ser Val Ile Ser
                245                 250                 255

Ser Leu Arg Arg Met Leu Leu Asn Trp Pro Ser Pro Pro Gly Ala Thr
            260                 265                 270

Arg Glu Ile Leu Glu Gln Leu Leu Thr Ala Leu Ala Ser Ser Gln Thr
        275                 280                 285

Asp Val Tyr Thr Val Ala Arg Ile Ile Ala Pro Leu Arg Pro Thr Asn
    290                 295                 300

Val Ala Asp Tyr Arg His Val Ala Phe Trp Gln Arg Leu Arg Tyr Phe
305                 310                 315                 320

Cys Arg Leu Tyr Leu Gln Ser Ser Gln Glu Leu His Arg Leu Gln Ser
                325                 330                 335

Gly Val Asp Asp His Thr Arg Leu Pro Arg Thr Ser Gly Leu Ala Arg
            340                 345                 350

His Thr Asp Asn Ala Glu Ala Met Trp Ser Gly Leu Arg Thr Phe Cys
        355                 360                 365

Thr Leu Met Met Ile Gly Ala Trp Ser Ile Ala Ser Gln Trp Asp Ala
    370                 375                 380

Gly Ala Asn Ala Leu Thr Leu Ala Ala Ile Ser Cys Val Leu Tyr Ser
385                 390                 395                 400

Ala Val Ala Ala Pro Phe Lys Ser Leu Ser Leu Leu Met Arg Thr Leu
                405                 410                 415

Val Leu Leu Ser Leu Phe Ser Phe Val Val Lys Phe Gly Leu Met Val
            420                 425                 430

Gln Ile Ser Asp Leu Trp Gln Phe Leu Leu Phe Leu Phe Pro Leu Leu
        435                 440                 445

Ala Thr Met Gln Leu Leu Lys Leu Gln Met Pro Lys Phe Ala Ala Leu
    450                 455                 460

Trp Gly Gln Leu Ile Val Phe Met Gly Ser Phe Ile Ala Val Thr Asn
465                 470                 475                 480

Pro Pro Val Tyr Asp Phe Ala Asp Phe Leu Asn Asp Asn Leu Ala Lys
                485                 490                 495

Ile Val Gly Val Ala Leu Ala Trp Leu Ala Phe Ala Ile Leu Arg Pro
            500                 505                 510

Gly Ser Asp Ala Arg Lys Ser Arg Arg His Ile Arg Ala Leu Arg Arg
        515                 520                 525

Asp Phe Val Asp Gln Leu Ser Arg His Pro Thr Leu Ser Glu Ser Glu
    530                 535                 540

Phe Glu Ser Leu Thr Tyr His His Val Ser Gln Leu Ser Asn Ser Gln
545                 550                 555                 560

Asp Ala Leu Ala Arg Arg Trp Leu Leu Arg Trp Gly Val Val Leu Leu
                565                 570                 575

Asn Cys Ser His Val Val Trp Gln Leu Arg Asp Trp Glu Ser Arg Ser
            580                 585                 590

Asp Pro Leu Ser Arg Val Arg Asp Asn Cys Ile Ser Leu Leu Arg Gly
        595                 600                 605

Val Met Ser Glu Arg Gly Val Gln Gln Lys Ser Leu Ala Ala Thr Leu
    610                 615                 620

Glu Glu Leu Gln Arg Ile Cys Asp Ser Leu Ala Arg His His Gln Pro
625                 630                 635                 640

Ala Ala Arg Glu Leu Ala Ala Ile Val Trp Arg Leu Tyr Cys Ser Leu
                645                 650                 655

Ser Gln Leu Glu Gln Ala Pro Pro Gln Gly Thr Leu Ala Ser
            660                 665                 670

<210>    39
<211>    1185
<212>    DNA
<213>    Artificial Sequence

<220>
<223>    ermD 1071G

<400>    39
atgaaaaggc aaagaaacgt caatttgtta ttgatgttgg tattactcgt ggccgtcggt      60

cagatggcgc aaaccattta tattccagct attgccgata tggcgcgcga tctcaacgtc     120

cgtgaagggg cggtgcagag cgtaatgggc gcttatctgc tgacttacgg tgtctcacag     180

ctgttttatg gcccgatttc cgaccgcgtg ggccgccgac cggtgatcct cgtcggaatg     240

tccattttta tgctggcaac gctggtcgcg gtcacgacct ccagtttgac ggtgttgatt     300

gccgccagcg cgatgcaggg gatgggcacc ggcgttggcg gcgtaatggc gcgtacttta     360

ccgcgagatt tatatgaacg gacacagttg cgccatgcta acagcctgtt aaacatgggg     420

attctcgtca gtccgttgct cgcaccgcta atcggcggtc tgctggatac gatgtggaac 480

tggcgcgcct gttatctctt tttgttggtt ctttgtgctg gtgtgacctt cagtatggcc 540

cgctggatgc cggaaacgcg tccggtcgat gcaccgcgca cgcgcctgct taccagttat 600

aaaacgcttt tcggtaacag cggttttaac tgttatttgc tgatgctgat tggcggtctg 660

gccgggattg ccgcctttga agcctgctcc ggcgtgctga tgggcgcggt gttagggctg 720

agcagtatga cggtcagtat tttgtttatt ctgccgattc cggcagcgtt ttttggcgca 780

tggtttgccg gacgtcccaa taaacgcttc tccacgttaa tgtggcagtc ggttatctgc 840

tgcctgctgg ctggcttgct gatgtggatc cccgactggt ttggcgtgat gaatgtctgg 900

acgctgctcg ttcccgccgc gctgttcttt ttcggtgccg ggatgctgtt tccgctggcg 960

accagcggcg cgatggagcc gttccccttc ctggcgggca cggctggcgc gctggtcggc 1020

ggtctgcaaa acattggttc cggcgtgctg gcgtcgctct ctgcgatgtt gccgcaaacc 1080

ggtcagggca gcctggggtt gttgatgacc ttaatgggat tgttgatcgt gctgtgctgg 1140

ctgccgctgg cgacgcggat gtcgcatcag gggcagcccg tttaa 1185